# EUROPEAN PATENT APPLICATION

(11) **EP 3 696 545 A1**
(43) Date of publication of application: **19.08.2020**
(21) Application number: 19305175.2
(22) Date of filing: 12.02.2019
(51) Int. Cl.: G01N 33/50, G01N 33/574, C12Q 1/6886

(54) **METHOD FOR PREDICTING THE RESISTANCE OF MELANOMA TO A TREATMENT WITH A BRAF INHIBITOR**

(71) Applicant: AnyGenes, 75970 Paris Cedex 20 (FR)
(72) Inventor: NAIMI, Ben-Youssef, 75007 Paris (FR)
(74) Representative: Gevers & Orès

(57) **Abstract**

The present invention relates to a method for predicting the resistance of a cancer to a treatment with a BRAF inhibitor alone or in combination with a MEK inhibitor. The invention further relates to a kit allowing the prediction of the resistance of a cancer to a combination treatment with a BRAF inhibitor, alone or in combination with a MEK inhibitor.

## Description

The present invention relates to a method for predicting the resistance of a cancer to a treatment with a BRAF inhibitor, alone or in combination with a MEK inhibitor. The invention further relates to a kit allowing the prediction of the resistance of a cancer to a treatment with a BRAF inhibitor alone or in combination with a MEK inhibitor.

Cancer remains a major cause of death in developed countries. In order to be most effective, cancer therapies require not only the early detection of the malignancy, but the reliable assessment of the likelihood of cancer resistance to conventional treatments. Currently, cancer therapies are often inefficient because methods are lacking for predicting the effectiveness of a treatment for a particular cancer's physiology. This may result in relatively poor response rates, increased cancer morbidity, relapse or death. Furthermore, patients often needlessly undergo ineffective, toxic drug therapy.

In particular, malignant melanoma is the most dangerous form of skin tumor and causes 90% of skin cancer mortality. It is the ninth most common cancer in Europe, with more than 100,000 new cases diagnosed in 2012. Melanoma has been one of the fastest rising malignancies in the last four decades with cases in the European Union countries increasing from below three per 100,000 people to above 15, with a mortality rate of 4 per 100,000, and the increase persists despite preventive efforts to educate the public about the dangers of excessive exposure to UV radiation. Melanoma is notorious for its tendency to metastasize and for its poor response to current therapeutic regimens. Metastatic melanoma has an extremely poor prognosis, with a median survival time of only 6.2 months from the diagnosis of distant metastases. Dacarbazine, an alkylating chemotherapy drug, which has been widely used as a first-line treatment of metastatic melanoma, achieves a partial response rate of only 7.5%. Thanks to fundamental research, targeted therapies were brought to clinic with a certain success.

The discovery of mutant *BRAF* gene as an important oncogenic driver, which is detected in approximately 60% of patients with metastatic melanoma (Davies et al., Mutations of the BRAF gene in human cancer. Nature. 2002;417:949-954), has led to the development of small molecule inhibitors of the BRAF kinase. Of these, vemurafenib and dabrafenib have been the most intensively studied (Deng et al., Role and therapeutic potential of PI3K-mTOR signaling in de novo resistance to BRAF inhibition (BRAFi). Pigment cell & melanoma research. 2012;25(2):248-58 ; Dummer & Flaherty, Resistance patterns with tyrosine kinase inhibitors in melanoma: new insights. Current opinion in oncology. 2012;24(2): 150-4). In preclinical experiments, both compounds inhibited MAPK signaling in cell lines harboring oncogenic *BRAF* and resulted in the inhibition of growth and survival of cells. Similarly encouraging results were also seen in xenograft models of BRAFV600E mutant melanoma and both drugs rapidly advanced into clinical trials. In large randomized phase III clinical trials, both vemurafenib and dabrafenib greatly improved response rate and increased progression-free-survival compared to dacarbazine. Despite these impressive results for both vemurafenib and dabrafenib, the majority of patients relapsed within a few months, due to the development of secondary resistance. Over the past several years, a substantial amount of research has focused on sorting out the mechanisms of resistance to first BRAF and more recently, to the inhibitors of BRAF downstream target MEK, in patients with BRAF-mutant melanoma.

Although the mechanisms of both intrinsic and acquired resistance are still being elucidated, several unique mechanisms have been identified. The mechanisms of resistance to BRAFi are multiple and complex and mostly described from *in vitro* experiments and more recently from *in vivo* studies. They include:
(i) a re-activation of the RAS-RAF-MEK-ERK pathway through RAS mutation, MEK, BRAF amplification, overexpression of COT or RAF protein isoforms, and differential splicing leading to truncated variants of BRAF or activation of MAPKK.
(ii) the activation of parallel pro-survival pathways frequently leading to activation of the PI3K pathway. Indeed, intrinsic and acquired BRAF inhibition resistances can be related with a marked activation of the PI3K-AKT pathway which includes loss of the PTEN tumor suppressor with subsequent suppression of BIM-mediated apoptosis, activation of the insulin-like growth factor-1 receptor (IGF1R) or other growth factors such as PDGFRb or HGF.
(iii) the expression of tyrosine kinase receptor CMET on tumor cells along with hepatocyte growth factor (CMET ligand) expression in the tumor microenvironment.

These intricate resistant mechanisms demonstrate a crucial need for a molecular tool that can detect and identify for a given patient the exact nature of his molecular resistance to BRAF inhibition (BRAFi) and MEK inhibition (MEKi) to enable an alternative therapeutic management.

The Inventors have in a surprising manner discovered a set of 22 genes, whose level of expression in a BRAF mutated melanoma model (tumor sample) allows the efficient detection of the resistances to a treatment with a BRAF inhibitor, alone or in combination with a MEK inhibitor. Advantageously, this minimal set of biomarkers allows the identification of distinct expression profiles associated to these resistances.

Thus, the present invention concerns an *in vitro* method for predicting the resistance of a cancer to a treatment with a BRAF inhibitor, alone or in combination with a MEK inhibitor, comprising:
- a step (i) of measuring the expression levels of a set of genes comprising at least the 22 biomarker genes *BRAF*, *RAF1*, *ARAF*, *PDGFRB*, *IGF1R*, *MET*, *EGFR*, *ERBB2*, *MAP3K8*, *MKI67*, *E2F2*, *CDK2*, *CDK4*, *CDK6*, *CCND1*, *DUSP4*, *DUSP6*, *BCL2*, *BCL2L1*, *BMF*, *BAD* and *SPRY4* in a tumor sample of said cancer.
- a step (ii) of determining the variations of each expression level provided in step (i) compared to a reference value,
- a step (iii) of determining whether the variations of each expression level provided in step (ii) are increased or decreased above a cut-off level,
wherein an increased or decreased expression level of at least one biomarker gene indicates that said cancer can be predicted as resistant to a treatment with a BRAF inhibitor, alone or in combination with a MEK inhibitor.

As used herein, the term "biomarker gene" refers to a differentially expressed gene, including all its isoform(s) and variant(s), whose expression pattern can be determined and correlated with a known condition, in particular with the presence of a resistance mechanism to an anti-cancerous treatment.

The 22 genes used as biomarkers in the present invention are given in Table 1.

**Table 1. List of the 22 biomarker genes.**

| **Gene** | **Definition** | **Accession Number** | **SEQ ID Number** |
|---|---|---|---|
| ***BRAF*** | v-raf murine sarcoma viral oncogene homolog B 1 | NG_007873.3 | **1** |
| ***RAF1*** | v-raf-1 murine leukemia viral oncogene homolog 1 | NG_007467.1 | **2** |
| ***ARAF*** | v-raf murine sarcoma 3611 viral oncogene homolog | NG_016339.2 | **3** |
| ***PDGFRB*** | platelet-derived growth factor receptor, beta polypeptide | NG_023367.1 | **4** |
| ***IGF1R*** | insulin-like growth factor 1 receptor | NG_009492.1 | **5** |
| ***MET*** | met proto-oncogene (hepatocyte growth factor receptor); | NG_008996.1 | **6** |
| ***EGFR*** | epidermal growth factor receptor | NG_007726.3 | **7** |
| ***ERBB2*** | v-erb-b2 erythroblastic leukemia viral oncogene homolog 2, neuro/glioblastoma derived oncogene homolog (avian); | NG_007503.1 | **8** |
| ***MAP3K8*** | mitogen-activated protein kinase kinase kinase 8 | NG_029984.1 | **9** |
| ***MK167*** | antigen identified by monoclonal antibody Ki-67 | NG_047061.1 | **10** |
| ***E2F2*** | E2F transcription factor 2 | NC_000001.11 | **11** |
| ***CDK2*** | cyclin-dependent kinase 2 | NG_034014.1 | **12** |
| ***CDK4*** | cyclin-dependent kinase 4 | NG_007484.2 | **13** |
| ***CDK6*** | cyclin-dependent kinase 6 | NG_015888.1 | **14** |
| ***CCND1*** | cyclin D1 | NG_007375.1 | **15** |
| ***BCL2*** | B-cell CLL/lymphoma 2 | NG_009361.1 | **16** |
| ***BCL2L1*** | BCL2-like 1 | NG_029002.1 | **17** |
| ***BMF*** | Bcl2 modifying factor | NC_000015.10 | **18** |
| ***BAD*** | BCL2-associated agonist of cell death | NC_000011.10 | **19** |
| ***SPRY4*** | sprouty RTK signaling antagonist 4 | NG_034148.1 | **20** |
| ***DUSP4*** | dual specificity phosphatase 4 | NC_000008.11 | **21** |
| ***DUSP6*** | dual specificity phosphatase 6 | NG_033915.1 | **22** |

In an embodiment, the reference value of step (ii) corresponds to the expression level of the same gene in the organ from which said tumor originates, in a benign tumor tissue corresponding to said cancer or in a normal cellular subtype of the organ from which said cancer originates.

In an embodiment, the cut-off level of step (iii) corresponds to at least a 2-fold, 3-fold, 4-fold, 5-fold, 6-fold, 7-fold, 8-fold, 9-fold or 10-fold increase or decrease of the expression level.

In a preferred embodiment, the cut-off level of step (iii) corresponds to at least a 2-fold increase or decrease of the expression level. In other words, a 2-fold increased or decreased level of at least one biomarker gene indicates that said cancer can be predicted as resistant to a treatment with a BRAF inhibitor, alone or in combination with a MEK inhibitor.

In an embodiment, the invention relates to the method as defined above, wherein increased or decreased expression levels of at least 2, 3, 4, 5, 6, 7, 8, 9, 10, 11, 12, 13, 14, 15, 16, 17, 18, 19, 20, 21 or 22 biomarker genes indicate that said cancer can be predicted as resistant to a treatment with a BRAF inhibitor, alone or in combination with a MEK inhibitor.

Advantageously, the method of the invention allows the detection and the identification of specific biomarker profiles (or signatures) which are associated to a resistance mechanism to BRAF, possibly in combination with MEK inhibition.

In an embodiment, the invention relates to the method as defined above, wherein the presence of an expression profile selected from the group comprising:
- profile 1: increased expression levels of *BRAF, CDK2, CDK4, CCND1*, *BCL2, BCL2L1*, *PDGFRB, MET* and *ERBB2* with a decreased expression level of *BMF,*
- profile 2: increased expression levels of *MAP3K8, DUSP4, DUSP6* and *EGFR* with a decreased expression level of *BAD*,
- profile 3: increased expression levels of *DUSP6*, *CCND1*, *IGF1R* and *PDGFRB* with a decreased expression level of *BMF,*
- profile 4: increased expression levels of *E2F2, MKI67, BCL2, MET, SPRY4* with decreased expression levels of *DUSP4, BCL2L1*,
- profile 5: increased expression levels of *RAF1, DUSP6, CDK6* and *ERBB2,*
- profile 6: increased expression levels of *BRAF, MKI67, CCND1*, *BCL2L1*, *IGF1R*, *MET* and *SPRY4* with a decreased expression level of *DUSP4,* and
- profile 7: increased expression levels of *ARAF*, *E2F2*, *CDK2*, *CCND1*, *BCL2*, *EGFR* and *MET* with decreased expression levels of *BAD* and *BCL2L1*,
   indicates that the cancer can be predicted as resistant to a treatment with a BRAF inhibitor, alone or in combination with a MEK inhibitor.

The expression level of genes can be determined by any method known by one skilled person in the art. In particular, expression level can be determined by measuring the quantity of mRNA and/or by measuring the quantity of encoded protein.

Methods for determining the quantity of mRNA are well known in the art and include, but are not limited to, quantitative or semi-quantitative RT-PCR, real time quantitative or semi-quantitative RT-PCR, Nanostring technology, sequencing based approaches or transcriptome approaches.

The nucleic acid contained in the tumor sample (e.g., cells or tissue prepared from the subject) may be first extracted according to standard methods, for example using lytic enzymes or chemical solutions or extracted by nucleic-acid-binding resins following the manufacturer's instructions. These nucleic acids may be frozen to be stored before use.

The extracted mRNA may be then detected by hybridization (e.g., Northern blot analysis) and/or amplification (e.g., RT-PCR). Quantitative or semi-quantitative RT-PCR is preferred. Real-time quantitative or semi-quantitative RT-PCR is particularly advantageous. Preferably, primer pairs were designed in order to overlap an intron, so as to distinguish cDNA amplification from putative genomic contamination. Such primers may be easily designed by the skilled person. Other methods of amplification include, but are not limited to, ligase chain reaction (LCR), transcription-mediated amplification (TMA), strand displacement amplification (SDA) and nucleic acid sequence based amplification (NASBA).

Alternatively, the quantity of mRNA may also be measured using the Nanostring's NCOUNTER™ Digital Gene Expression System (Nanostring Technologies) which captures and counts individual mRNA transcripts by a molecular bar-coding technology, or the QuantiGene® Plex 2.0 Assay (Affymetrix). The quantity of mRNA may further be determined using approaches based on high-throughput sequencing technology or sequencing technologies using microfluidic systems.

The expression level of a gene may also be determined by measuring the quantity of mRNA by transcriptome approaches, in particular by using DNA microarrays. To determine the expression level of a gene, the sample, optionally first subjected to a reverse transcription, is labelled and contacted with the microarray in hybridization conditions, leading to the formation of complexes between target nucleic acids that are complementary to probe sequences attached to the microarray surface. The labelled hybridized complexes are then detected and can be quantified or semi-quantified. Labelling may be achieved by various methods, e.g. by using radioactive or fluorescent labelling. Many variants of the microarray hybridization technology are available to the man skilled in the art.

Next Generation Sequencing methods (NGS) may also be used.

Methods for measuring the quantity or the activity of the encoded protein are also well-known by the skilled person and the choice of the method depends on the encoded protein. Usually, these methods comprise contacting the sample with a binding partner capable of selectively interacting with the protein present in the sample. The binding partner is generally a polyclonal or monoclonal antibody, preferably monoclonal. The quantity of protein is measured by semi-quantitative Western blots, immunochemistry (enzyme-labeled and mediated immunoassays, such as ELISAs, biotin/avidin type assays, radioimmunoassay, Immunoelectrophoresis or immunoprecipitation) or by protein or antibody arrays. In a particular embodiment, the protein expression level is assessed by reverse-phase protein microarray (RPPM). The protein expression level may also be assessed by immunohistochemistry on a tissue section of the cancer sample (e.g., frozen or formalin-fixed paraffin embedded material). The reactions generally include revealing labels such as fluorescent, chemiluminescent, radioactive, enzymatic labels or dye molecules, or other methods for detecting the formation of a complex between the antigen and the antibody or antibodies reacted therewith. Specific activity assays may also be used, in particular when the encoded protein is an enzyme.

In preferred embodiments, the expression levels of genes of step (i) are determined by measuring the quantity of mRNA by quantitative RT-PCR.

Preferably, expression levels of genes provided in step (i) and/or determined as described above, are normalized to a control expression level, preferably to the expression level of one or more control genes.

As used herein, the term "control gene" designates a gene that is transcribed at a relative constant level and thus used to normalize expression levels of genes that vary across different samples. Such control genes are preferably housekeeping genes, *i.e.* genes that are involved in basic functions needed for maintenance of the cell. Examples of such housekeeping genes include, but are not limited to *PPIA*, *ACTB*, *TBP*, *B2M*, *HPRT1* and *TFRC.*

In an embodiment, the set of genes further comprises at least one control gene selected from the group comprising *PPIA, ACTB, TBP, B2M, HPRT1* and *TFRC.*

The six genes used as control genes in the present invention are given in Table 2.

**Table 2. List of control genes.**

| **Gene** | **Definition** | **Accession Number** | **SEQ ID Number** |
|---|---|---|---|
| ***PPIA*** | peptidylprolyl isomerase A (cyclophilin A) | NG_029697.1 | **23** |
| ***ACTB*** | actin, beta | NG_007992.1 | **24** |
| ***TBP*** | TATA box binding protein | NG_008165.1 | **25** |
| ***B2M*** | beta-2-microglobulin | NG_012920.2 | **26** |
| ***HPRT1*** | Hypoxanthine guanine phosphoribosyl transferase I | NG_012329.2 | **27** |
| ***TFRC*** | Transferrin receptor (p90, CD71) | NG_046395.1 | **28** |

Means suitable for determining the expression levels of the biomarker genes and the control genes of the invention can be primers and/or probe specific to said genes.

**Table 3. List of primers and probes specific for the 22 biomarker genes and 6 control genes.**

| **Target gene** | **Forward Primer Sequence (SEQ ID Number)** | **Reverse Primer Sequence (SEQ ID Number)** | **Probe Primer Sequence (SEQ ID Number)** |
|---|---|---|---|
| ***BRAF*** | GTGAGGGCTCCAGCT TGTAT (SEQ ID NO: 29) | GGATGATTGACTTGGC GTGTA (SEQ ID NO: 30) | CGACAGACTGCACAGGGC ATGGAT (SEQ ID NO: 85) |
| ***RAF1*** | TCTTGCCGAACAAGC AAAG (SEQ ID NO: 31) | GCTTTCATAAGGCAGT CATGC (SEQ ID NO: 32) | CAGTGGTCAATGTGCGAA ATGGAATGA (SEQ ID NO: 86) |
| ***ARAF*** | GTGTGGTCTACCGAC TCATCA (SEQ ID NO: 33) | GTCAGCGGGACATCTT CAA (SEQ ID NO: 34) | AGGGACGAAAGACGGTCA CTGCC (SEQ ID NO: 87) |
| ***PDGFRB*** | CCTTCCATGAGGATG CTGAG (SEQ ID NO: 35) | TCAGGGTGGCTCTCAC TTAG (SEQ ID NO: 36) | TCTCCTTCCAGCTACAGA TCAATGTCCCT (SEQ ID NO: 88) |
| ***IGF1R*** | GGTGGAGAACGACCA TATCC (SEQ ID NO: 37) | TGAGAAGAGGAGTTCG ATGC (SEQ ID NO: 38) | AGTTCCTTCCATTCCCTT GGACGTTCTT (SEQ ID NO: 89) |
| ***MET*** | ACTCCCATCCAGTGT CTCCA (SEQ ID NO: 39) | GCAGCCCAAGCCATTC A (SEQ ID NO: 40) | CTGGTTATCACTGGGAAG AAGATCACGAAGAT (SEQ ID NO: 90) |
| ***EGFR*** | AAAGTGTGATCCAAG CTGTCC (SEQ ID NO: 41) | ACTGGTTGTGGCAGCA GTC (SEQ ID NO: 42) | TGCAGGAGAGGAGAACTG CCAGAAACT (SEQ ID NO: 91) |
| ***ERBB2*** | AGACACGTTTGAGTC CATGC (SEQ ID NO: 43) | CTGTCACCTCTTGGTT GTGC (SEQ ID NO: 44) | CCCTACAACTACCTTTCT ACGGACGTGGG (SEQ ID NO: 92) |
| ***MAP3K8*** | TGGCACAAGATATAA AGACGAA (SEQ ID NO: 45) | CGGAAGCAAGCCTGGA T (SEQ ID NO: 46) | AAAGAATGGCGTGTAAAC TGATCCCAG (SEQ ID NO: 93) |
| ***MKI67*** | CACACTCCACCTGTC CTGAA (SEQ ID NO: 47) | TTTGTGCCTTCACTTC CACA (SEQ ID NO: 48) | CAAGGAACAGCCTCAACC ATCAGGAAA (SEQ ID NO: 94) |
| ***E2F2*** | CAGGAGCTGAAGGAG CTGA (SEQ ID NO: 49) | TGCCAACAGCACGGAT A (SEQ ID NO: 50) | ACTGAGGACAAGGCCAAC AAGAGGC (SEQ ID NO: 95) |
| ***CDK2*** | AGATCGGAGAGGGCA CGTA (SEQ ID NO: 51) | TCAGTGTCCAGGCGGA TT (SEQ ID NO: 52) | AAGTTGACGGAGAGGTG GTGGC (SEQ ID NO: 96) |
| ***CDK4*** | CAGTTCGTGAGGTGG CTTTAC (SEQ ID NO: 53) | GGTCAGTTCGGGATGT GG (SEQ ID NO: 54) | CATCCCAATGTTGTCCGG CTGATG (SEQ ID NO: 97) |
| ***CDK6*** | ATGCCGCTCTCCACC ATC (SEQ ID NO: 55) | GGTTTCTCTGTCTGTT CGTGAC (SEQ ID NO: 56) | CGTGGTCAGGTTGTTTGA TGTGTGC (SEQ ID NO: 98) |
| ***CCND1*** | CCCTCGGTGTCCTAC TTCA (SEQ ID NO: 57) | AAGACCTCCTCCTCGC ACT (SEQ ID NO: 58) | ATGCTGGAGGTCTGCGAG GAACA (SEQ ID NO: 99) |
| ***BCL2*** | CTGGGATGCCTTTGT GGAA (SEQ ID NO: 59) | CAGCCAGGAGAAATCA AACAGA (SEQ ID NO: 60) | TGTACGGCCCCAGCATGC G (SEQ ID NO: 100) |
| ***BCL2L1*** | CTGGTGGTTGACTTT CTCTCC (SEQ ID NO: 61) | GGTCTCCATCTCCGAT TCAG (SEQ ID NO: 62) | TAGTGATGTGGAAGAGAA CAGGACTGAGGC (SEQ ID NO: 101) |
| ***BMF*** | TGTGTTCCAACCAGA GGATG (SEQ ID NO: 63) | CAGTGGGTGAGAGGGA AGAG (SEQ ID NO: 64) | TGCTGACCTGTTTGCCCA GAGCCTA (SEQ ID NO: 102) |
| ***BAD*** | GATGAGTGACGAGTT TGTGGAC (SEQ ID NO: 65) | AAGTTCCGATCCCACC AG (SEQ ID NO: 66) | CCTTTAAGAAGGGACTTC CTCGCCCG (SEQ ID NO: 103) |
| ***SPRY4*** | TGGGAAGTGTAAATG CAAGG (SEQ ID NO: 67) | CGTGCCATAGTTGACC AGAG (SEQ ID NO: 68) | TCTGCAACCAGGAGTGCC TGTGCT (SEQ ID NO: 104) |
| ***DUSP4*** | TCAGTACAAGTGCAT CCCAGT (SEQ ID NO: 69) | AGTCCTTCACGGCATC G (SEQ ID NO: 70) | AAGATAACCACAAGGCCG ACATCAGCTC (SEQ ID NO: 105) |
| ***DUSP6*** | GAAATGGCGATCAGC AAGA (SEQ ID NO: 71) | GACTCGATGTGCGACG ACT (SEQ ID NO: 72) | CGGCTGCTGCTGATGGAC TGC (SEQ ID NO: 106) |
| ***PPIA*** | GTCAACCCCACCGTG TTCTT (SEQ ID NO: 73) | CTGCTGTCTTTGGGAC CTTGT (SEQ ID NO: 74) | CGCGTCTCCTTTGAGCTG TTTGCAG (SEQ ID NO: 107) |
| ***ACTB*** | CTCCTCCTGAGCGCA AGTA (SEQ ID NO: 75) | CCGGACTCGTCATACT CCTG (SEQ ID NO: 76) | CTCGCTGTCCACCTTCCA GCAGAT (SEQ ID NO: 108) |
| ***TBP*** | CACGAACCACGGCAC TGATT (SEQ ID NO: 77) | TTTCTTGCTGCCAGTC TGGAC (SEQ ID NO: 78) | TGCACAGGAGCCAAGAGT GAAGAACA (SEQ ID NO: 109) |
| ***B2M*** | TCCAGCGTACTCCAA AGATTC (SEQ ID NO: 79) | GACAAGTCTGAATGCT CCACTT (SEQ ID NO: 80) | CTATGTGTCTGGGTTTCA TCCATCCGAC (SEQ ID NO: 110) |
| ***HPRT1*** | TTCCTTGGTCAGGCA GTATAATC (SEQ ID NO: 81) | GGCTTATATCCAACAC TTCGTG (SEQ ID NO: 82) | AAAGATGGTCAAGGTCGC AAGCTTGC (SEQ ID NO: 111) |
| ***TFRC*** | CAGCAAAGTCTGGCG TGA (SEQ ID NO: 83) | CCGTTCTTATCAACTA TGATCACC (SEQ ID NO: 84) | TTAAGATTCAGGTCAAAG ACAGCGCTC (SEQ ID NO: 112) |

By "sample" is meant, according to the invention, any sample containing cells obtained from a subject, preferably containing nucleic acids. Examples of such samples include fluids, such as blood and plasma samples, as well as biopsies, organs, tissues or cell samples. Such a sample may be treated prior to its use.

The term "tumor sample" refers to any sample containing tumoral cells obtained from a subject. Preferably, the sample contains only tumoral cells.

In preferred embodiments, the tumor sample is a biopsy or is derived from a biopsy obtained from a subject during surgery.

As used herein, the term "subject" refers to an animal, preferably to a mammal, even more preferably to a human, including adult, child and human at the prenatal stage.

The term "cancer" as used herein, refers to the presence of cells possessing characteristics typical of cancer-causing cells, such as uncontrolled proliferation, immortality, metastatic potential, rapid growth and proliferation rate, and certain characteristic morphological features. This term includes early stage, localized, cancer; later stage, locally advanced cancer; and metastatic stage cancer.

In most preferred embodiments, cancer cells have the BRAF V600 mutation, which constitutively activates the MAPK/ERK pathway.

In preferred embodiments, the cancer is selected from the group consisting of skin cancer, colorectal cancer, intestine cancer, prostate cancer, lung cancer, pancreas cancer, liver cancer, genital cancer, kidney cancer, thyroid cancer, pituitary cancer, central nervous system cancer, stomach cancer, bone cancer, head and neck cancer, breast cancer, glioma and hematologic cancer.

Even more preferably, the cancer is selected from skin cancer, colon cancer and a hematologic cancer
As used herein, the term "skin cancer" refers to cancer arising in the skin of any histological type, including but not limited to basal cell carcinoma, Merkel cell carcinoma, squamous cell carcinoma or melanoma. Preferably, the skin cancer is melanoma.

As used herein, the term "colorectal cancer" refers to a cancer arising in the large intestine (including both the colon and rectum) of any histologic type, including but not limited to malignant epithelial tumors. The colorectal cancer may be adenocarcinoma, carcinoid tumor, mucinous adenocarcinoma (also termed colloid adenocarcinoma), signet ring adenocarcinoma, scirrhous tumor, carcinoma simplex or sarcoma. Preferably, the colon cancer is adenocarcinoma.

As used herein, the term "hematological cancer" (or blood cancer) refers to a cancer that begins in blood-forming tissue, such as the bone marrow, or in the cells of the immune system. Examples of hematologic cancer are leukemia, lymphoma, and multiple myeloma. Preferably, the cancer is a leukemia, more preferably, a hairy cell leukemia.

As used herein, the term "BRAF Inhibitor" refers to any compound that, directly or indirectly, inhibits the activity of the serine/threonine protein kinase B-Raf.

In an embodiment, BRAF inhibitor is selected from the group comprising vemurafenib, dabrafenib, encorafenib and sorafenib, preferably vemurafenib.

As used herein, the term "MEK inhibitor" refers to any compound that, directly or indirectly, inhibits the activity of the mitogen-activated protein kinase kinase.

In an embodiment, MEK inhibitor is selected from the group comprising cobimetinib, trametinib, pimasertib and binimetinib, preferably cobimetinib.

In preferred embodiments, BRAF inhibitor is vemurafenib and MEK inhibitor is cobimetinib.

Another aspect of the invention relates to a kit comprising primers, probes or antibodies specific to at least the 22 biomarker genes *BRAF, RAF1, ARAF, PDGFRB, IGF1R*, *MET, EGFR, ERBB2, MAP3K8, MKI67, E2F2, CDK2, CDK4, CDK6, CCND1*, *DUSP4, DUSP6, BCL2, BCL2L1*, *BMF, BAD* and *SPRY4,* and optionally, a leaflet providing guidelines to use such a kit.

In an embodiment, the kit according to the invention further comprises primers, probes or antibodies specific to at least one control gene selected from the group comprising *PPIA, ACTB, TBP, B2M, HPRT1* and *TFRC.*

In preferred embodiments, the kit according to the invention comprises an array wherein probes specific of the biomarker genes and/or the control genes as described above, are immobilized.

Such primer and probes may be easily designed by the skilled person.

In an embodiment, the kit according to the invention comprises at least one pair of primers selected from the group comprising SEQ ID NO: 29/30, SEQ ID NO: 31/32, SEQ ID NO: 33/34, SEQ ID NO: 35/36, SEQ ID NO: 37/38, SEQ ID NO: 39/40, SEQ ID NO: 41/42, SEQ ID NO: 43/44, SEQ ID NO: 45/46, SEQ ID NO: 47/48, SEQ ID NO: 49/50, SEQ ID NO: 51/52, SEQ ID NO: 53/54, SEQ ID NO: 55/56, SEQ ID NO: 57/58, SEQ ID NO: 59/60, SEQ ID NO: 61/62, SEQ ID NO: 63/64, SEQ ID NO: 65/66, SEQ ID NO: 67/68, SEQ ID NO: 69/70 and SEQ ID NO: 71/72.

In an embodiment, the kit according to the invention comprises at least 2, 3, 4, 5, 6, 7, 8, 9, 10, 11, 12, 13, 14, 15, 16, 17, 18, 19, 20 or 22 pairs of primers selected from the group comprising SEQ ID NO: 29/30, SEQ ID NO: 31/32, SEQ ID NO: 33/34, SEQ ID NO: 35/36, SEQ ID NO: 37/38, SEQ ID NO: 39/40, SEQ ID NO: 41/42, SEQ ID NO: 43/44, SEQ ID NO: 45/46, SEQ ID NO: 47/48, SEQ ID NO: 49/50, SEQ ID NO: 51/52, SEQ ID NO: 53/54, SEQ ID NO: 55/56, SEQ ID NO: 57/58, SEQ ID NO: 59/60, SEQ ID NO: 61/62, SEQ ID NO: 63/64, SEQ ID NO: 65/66, SEQ ID NO: 67/68, SEQ ID NO: 69/70 and SEQ ID NO: 71/72.

In an embodiment, the kit according to the invention comprises at least one pair of primers selected from the group comprising SEQ ID NO: 29/30, SEQ ID NO: 31/32, SEQ ID NO: 33/34, SEQ ID NO: 35/36, SEQ ID NO: 37/38, SEQ ID NO: 39/40, SEQ ID NO: 41/42, SEQ ID NO: 43/44, SEQ ID NO: 45/46, SEQ ID NO: 47/48, SEQ ID NO: 49/50, SEQ ID NO: 51/52, SEQ ID NO: 53/54, SEQ ID NO: 55/56, SEQ ID NO: 57/58, SEQ ID NO: 59/60, SEQ ID NO: 61/62, SEQ ID NO: 63/64, SEQ ID NO: 65/66, SEQ ID NO: 67/68, SEQ ID NO: 69/70, SEQ ID NO: 71/72, SEQ ID NO: 73/74, SEQ ID NO: 75/76, SEQ ID NO: 77/78, SEQ ID NO : 79/80, SEQ ID NO: 81/82 and SEQ ID NO: 83/84.

In an embodiment, the kit according to the invention comprises at least 2, 3, 4, 5, 6, 7, 8, 9, 10, 11, 12, 13, 14, 15, 16, 17, 18, 19, 20, 22, 23, 24, 25, 26, 27 or 28 pairs of primers selected from the group comprising SEQ ID NO: 29/30, SEQ ID NO: 31/32, SEQ ID NO: 33/34, SEQ ID NO: 35/36, SEQ ID NO: 37/38, SEQ ID NO: 39/40, SEQ ID NO: 41/42, SEQ ID NO: 43/44, SEQ ID NO: 45/46, SEQ ID NO: 47/48, SEQ ID NO: 49/50, SEQ ID NO: 51/52, SEQ ID NO: 53/54, SEQ ID NO: 55/56, SEQ ID NO: 57/58, SEQ ID NO: 59/60, SEQ ID NO: 61/62, SEQ ID NO: 63/64, SEQ ID NO: 65/66, SEQ ID NO: 67/68, SEQ ID NO: 69/70, SEQ ID NO: 71/72, SEQ ID NO: 73/74, SEQ ID NO: 75/76, SEQ ID NO: 77/78, SEQ ID NO : 79/80, SEQ ID NO: 81/82 and SEQ ID NO: 83/84.

In an embodiment, the kit according to the invention comprises at least one probe selected from SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105 and SEQ ID NO: 106.

In an embodiment, the kit according to the invention comprises at least 2, 3, 4, 5, 6, 7, 8, 9, 10, 11, 12, 13, 14, 15, 16, 17, 18, 19, 20, 21 or 22 probes selected from SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105 and SEQ ID NO: 106.

In an embodiment, the kit according to the invention comprises at least one probe selected from SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111 and SEQ ID NO: 112.

In an embodiment, the kit according to the invention comprises at least 2, 3, 4, 5, 6, 7, 8, 9, 10, 11, 12, 13, 14, 15, 16, 17, 18, 19, 20, 21, 2, 23, 24, 25, 26, 27 or 28 probes selected from SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111 and SEQ ID NO: 112.

The kit according to the invention may further comprise additional reagents such as buffer(s), enzyme(s) or nucleotides.

Another aspect of the invention relates to the use of a kit as defined above for predicting the resistance of a cancer to a treatment with a BRAF inhibitor, alone or in combination with a MEK inhibitor, in a tumor sample of said cancer.

All embodiments disclosed for the methods of the invention are also contemplated in this aspect.

The following Figures and Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURES

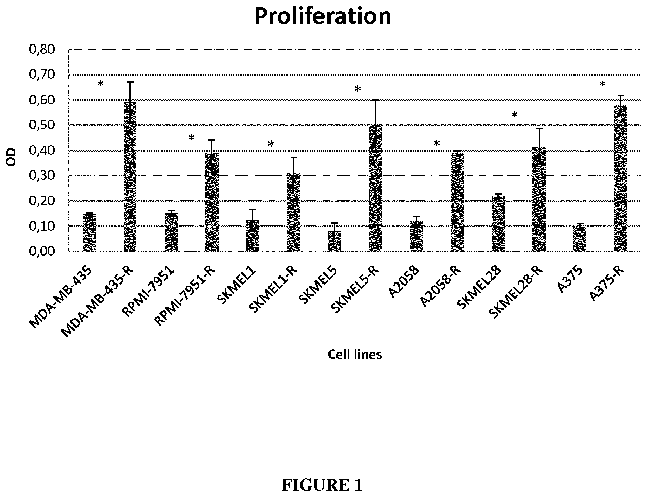

**Figure 1****.** Proliferation assay in native and resistant cell lines, MDA-MB-435, RPMI-7951, SKMEL1, SKMEL5, A2058, SKMEL28 and A375, treated with vemurafenib/cobimetinib. Bars represent means from three independent experiments. * p<0.05 (OD: Optical density).

### EXAMPLES

### Materials and Methods

### Chemicals

BRAF inhibitor, *i.e.* vemurafenib, and MEK inhibitor, *i.e.* cobimetinib, were purchased from Selleckchem (Houston, TX, USA).

### Cell lines culture

MDA-MB-435, RPMI-7951, SKMEL1, A375, SKMEL5, SKMEL28 and A2058 cell lines were obtained from American Type Culture Collection (ATCC, (Manassas, VA)). The seven cell lines correspond to distinct melanoma models with different genotypes and phenotypes. Cells were cultured in DMEM or RPMI supplemented with 10% Fetal Bovine Serum (FBS), 100 U/ml penicillin and 100 mg/ml streptomycin, at 37°C in a humidified incubator with 5% CO₂. Cells were cultured in DMEM medium. Vemurafenib/Cobimetinib resistant melanoma cells were chronically selected by culturing cells in increasing concentrations of drug association for at least four months. The selected resistant cells (cell line-R) increased vemurafenib/cobimetinib IC50 compared to the parental cells. Cells were maintained in full growth medium containing 2.5µM vemurafenib and 2µM of cobimetinib.

### MAPK/Proliferation/Apoptosis transcript expression study

mRNA expression analysis was performed by quantitative PCR (qPCR) using the personalized Human qPCR SignArrays® 96 system (qPCR SignArrays® kit, AnyGenes, France). A total volume of 20µl PCR mix, including 10µl of Perfect MasterMix SYBR Green®, 8µl of PCR grade water and 2µl of cDNA was dropped into each well of the qPCR array and analyzed with LightCycler 480 (Roche, France). PCR amplification was conducted in duplicates at 95°C for 10 minutes, followed by 40 cycles of 95°c for 10 seconds and 60°c for 30 seconds. Transcript expression analysis was performed on 22 genes of interest.

mRNA expression analysis was performed after reverse transcription and normalization using the expression of 6 housekeeping genes (peptidylprolyl isomerase A (cyclophilin A, *PPIA*), b-actin (*ACTB*), TATA box binding protein (*TBP*)*,* beta- 2-microglobulin (*B2M*), hypoxanthine phosphoribosyltransferase 1 (*HPRT1*) and transferring receptor (p90, CD71) *(TFRC).* mRNA expression for each gene was expressed as the ratio: copy number of gene of interest / copy number of at least one housekeeping gene.

Change of mRNA expression in each cell line-R were expressed as fold change between resistance and baseline cell line. A change ≥ 2 or ≤ 0.5 was considered respectively as a significant increase or decrease of mRNA expression.

### Proliferation assay

Cell proliferation was performed on native and resistant cell lines treated with vemurafenib/cobimetinib. Cell number was measured using the CellTiter 96 aqueous nonradioactive cell proliferation assay (Promega, France). All conditions were evaluated in triplicates.

### EXAMPLE 1 - Proliferation rate of native and resistant cell lines

Cell proliferation performed on native and resistant cell lines treated with vemurafenib/cobimetinib revealed an increase in proliferation rate in the 7 resistant cell models compared to matched native sensitive cells.

Indeed, a mean of 2-fold increase was observed in RPMI-7951- R, SKMEL1-R and SKMEL28-R cells while, 3-, 4- and 6-fold increase was observed in A2058-R, MDA-MB-435-R, A375-R and SKMEL5-R resistant cell lines respectively (Figure 1).

### EXAMPLE 2 - MAPK/Proliferation/Apoptosis transcript expression study

mRNA expression analysis was performed by qRT-PCR using personalized Human qPCR SignArrays® 96 kit. Transcripts levels were measured in native and resistant cells. Data analysis was conducted following two steps: first, transcript normalization was performed using a panel of 6 housekeeping genes. Then, each transcript fold change was expressed as the ratio of transcript expression between resistant cells/native cells.

For each cell line, as shown in Table 4, transcript expression profile as depicted by number of increased and deceased analyzed genes was as follows:
- MDA-MB-435: increase of 9 transcripts and decrease of 1 transcript (profile 1)
- A2058: increase of 7 transcripts and decrease of 1 transcript (profile 2)
- SKMEL-28: increase of 7 transcripts and decrease of 2 transcripts (profile 3)
- RPMI-7951: increase of 4 transcripts and decrease of 1 transcript (profile 4)
- A375: increase of 5 transcripts and decrease of 2 transcripts (profile 5)
- SKMEL-1: increase of 4 transcripts and decrease of 1 transcript (profile 6)
- SKMEL-5: increase of 4 transcripts (profile 7).

**Table 4. Differential expression of transcript levels between resistant cell line and native cell line. An increase of at least 2-fold (ratio > 2) is indicated in bold. A decrease of at least 2-fold (ratio < 0,5) is indicated by underscore. Blank cells indicate no change fold.**

| | MDA-MB-435 | RPMI-7951 | SKMEL-1 | A375 | SKMEL-5 | A2058 | SKMEL-28 |
|---|---|---|---|---|---|---|---|
| *BRAF* | **4,84** | | | | | **2,11** | |
| *RAF1* | | | | | **2,19** | | |
| *ARAF* | | | | | | | **2,70** |
| *MAP3K8* | | **5,20** | | | | | |
| *E2F2* | | | | **6,80** | | | **3,70** |
| *DUSP6* | | **3,48** | **2,61** | | **6,66** | | |
| *DUSP4* | | **3,52** | | 0,27 | | 0,09 | |
| *MKI67* | | | | **3,09** | | **6,43** | |
| *CDK2* | **2,61** | | | | | | **3,16** |
| *CDK4* | **2,92** | | | | | | |
| *CDK6* | | | | | **2,43** | | |
| *CCND1* | **4,69** | | **2,96** | | | **30,91** | **4,80** |
| *BCL2* | **4,92** | | | **3,09** | | | **3,14** |
| *BMF* | 0,34 | | 0,21 | | | | |
| *BAD* | | 0,45 | | | | | 0,34 |
| *BCL2L1* | **3,08** | | | 0,42 | | **2,30** | 0,22 |
| *EGFR* | | **6,76** | | | | | **2,22** |
| *IGF1R* | | | **4,71** | | | **8,70** | |
| *PDGFRB* | **2,30** | | **2,65** | | | | |
| *MET* | **22,15** | | | **3,87** | | **4,12** | **2,90** |
| *ERBB2* | **6,59** | | | | **8,51** | | |
| *SPRY4* | | | | **4,44** | | **8,32** | |

Interestingly, a variation is observed in one cell model for *RAF1, CDK6* and *CDK4*; and in four cell models for *CCND1*, *MET* and *BCL2L1.* The following genes variated in two different cell models: *BRAF, E2F, MKI67, CDK2, BMF, BAD, EGFR, SPRY4, IGF1R*, *ERBB2* and *PDGFRB.* Finally, *BCL2, DUSP4* and *DUSP6* variated in 3 different resistant models.

Taken together, the information obtained with the combination of the 22 selected transcripts reveals complementary profiles associated with resistant phenotypes of the seven studied cell lines.

## Claims

1. An *in vitro* method for predicting the resistance of a cancer to a treatment with a BRAF inhibitor, alone or in combination with a MEK inhibitor, comprising:
- a step (i) of measuring the expression levels of a set of genes comprising at least the 22 biomarker genes *BRAF, RAF1, ARAF, PDGFRB, IGF1R*, *MET, EGFR, ERBB2, MAP3K8, MKI67, E2F2, CDK2, CDK4, CDK6, CCND1*, *DUSP4, DUSP6, BCL2, BCL2L1*, *BMF, BAD* and *SPRY4* , in a tumor sample of said cancer.
- a step (ii) of determining the variations of each expression level provided in step (i) compared to a reference value,
- a step (iii) of determining whether the variations of each expression level provided in step (ii) are increased or decreased above a cut-off level,
wherein an increased or decreased expression level of at least one biomarker gene indicates that said cancer can be predicted as resistant to a treatment with a BRAF inhibitor, alone or in combination with a inhibitor.

2. The method according to claim 1, wherein said reference value corresponds to the expression level of the same gene in the organ from which said tumor originates, in a benign tumor tissue corresponding to said cancer or in a normal cellular subtype of the organ from which said cancer originates.

3. The method according to any one of claims 1-2, wherein said set of genes further comprises at least one control gene selected from the group comprising *PPIA, ACTB, TBP, B2M, HPRT1* and *TFRC.*

4. The method according to any one of claims 1-3, wherein said cancer is selected from the group consisting of skin cancer, colorectal cancer, intestine cancer, prostate cancer, lung cancer, pancreas cancer, liver cancer, genital cancer, kidney cancer, thyroid cancer, pituitary cancer, central nervous system cancer, stomach cancer, bone cancer, head and neck cancer, breast cancer, glioma and hematologic cancer, preferably a skin cancer, a colorectal cancer or an hematologic cancer.

5. The method according to any one of claims 1-4, wherein said cancer is a melanoma, an adenocarcinoma or a hairy cell leukemia

6. The method according to any one of claims 1-5, wherein said BRAF inhibitor is selected from the group comprising vemurafenib, dabrafenib, encorafenib and sorafenib, preferably vemurafenib.

7. The method according to any one of claims 1-6, wherein said MEK inhibitor is selected from the group comprising cobimetinib, trametinib, pimasertib and binimetinib, preferably cobimetinib.

8. The method according to any one of claims 1-7, wherein said tumor is obtained from a mammal, preferably a human.

9. The method according to any one of claims 1-8, wherein the cut-off level of step (iii) corresponds to at least a 2-fold increase or decrease of the expression level.

10. The method according to any one of claims 1-9, wherein increased or decreased expression levels of at least 2, 3, 4, 5, 6, 7, 8, 9, 10, 11, 12, 13, 14, 15, 16, 17, 18, 19, 20, 21 or 22 bio-marker genes indicate that said cancer can be predicted as resistant to a treatment with a BRAF inhibitor, alone or in combination with a MEK inhibitor.

11. A kit comprising primers, probes or antibodies specific to at least the 22 biomarker genes *BRAF, RAF1, ARAF, PDGFRB, IGF1R*, *MET, EGFR, ERBB2,* MAP3K8, *MKI67, E2F2, CDK2, CDK4, CDK6, CCND1*, *BCL2, BCL2L1*, *BMF, BAD,* SPRY4, *DUSP4* and *DUSP6.*

12. The kit according to claim 11, further comprising primers, probes or antibodies specific to at least one control gene selected from the group comprising *PPIA, ACTB, TBP, B2M, HPRT1* and *TFRC.*

13. The kit according to any one of claims 11-12 comprising an array wherein probes specific of said biomarker genes and/or said control genes are immobilized.

14. Use of a kit as defined in any one of claims 11-13 for predicting the resistance of a cancer to a treatment with a BRAF inhibitor, alone or in combination with a MEK inhibitor, in a tumor sample of said cancer.
